(19) Europäisches Patentamt
European Patent Office
Office européen des brevets

(11) Publication number: **0 157 637 A2**

# (12) EUROPEAN PATENT APPLICATION

(21) Application number: **85302321.6**

(22) Date of filing: **02.04.85**

(51) Int. Cl.⁴: **C 07 D 473/34**
// C07D473/40, C07D473/24, A61K31/52

(30) Priority: **04.04.84 GB 8408615**

(43) Date of publication of application: **09.10.85 Bulletin 85/41**

(84) Designated Contracting States: **AT BE CH DE FR GB IT LI LU NL SE**

(71) Applicant: **THE WELLCOME FOUNDATION LIMITED, 183-193 Euston Road, London NW1 2BP (GB)**

(72) Inventor: **Kelley, James Leroy, 10928 Raven Rock Drive, Raleigh North Carolina 27614 (US)**

(74) Representative: **Rollins, Anthony John et al, Group Patents & Agreements The Wellcome Foundation Ltd Langley Court, Beckenham Kent BR3 3BS (GB)**

(54) **Heterocyclic compounds.**

(57) The present invention provides a group of novel compounds useful in the treatment of CNS disorders such as epilepsy, the compounds having the general formula (III):

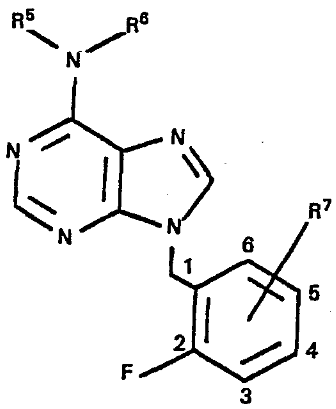

wherein $R^5$ is hydrogen, methyl or ethyl; $R^6$ is hydrogen, methyl, hydroxymethyl, methoxymethyl, ethyl, n-propyl, isopropyl, cyclopropyl, cyclopentyl, or cyclopropylmethyl and $R^7$ is hydrogen or fluorine attached to the 6- or 5- position of the phenyl ring.

Also provided are methods for the preparation of compounds of the formula (III) and pharmaceutical compositions containing the said compounds.

EP 0 157 637 A2

# HETEROCYCLIC COMPOUNDS

The present invention relates to a group of novel compounds which are useful in the treatment of CNS disorders, such as epilepsy, to pharmaceutical compositions containing them, and to methods for their preparation and to methods of treating CNS disorders, such as epilepsy, in mammals.

U.S. Patent No. 4189485 discloses, inter alia, that compounds of the formula (I):

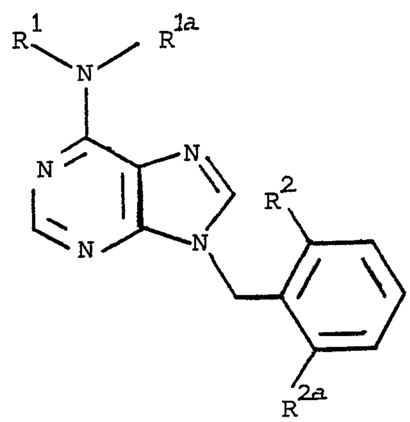

(I)

wherein $R^1$ is hydrogen or $C_{1-3}$ alkyl, $R^{1a}$ is $C_{1-3}$ alkyl or allyl, and $R^2$ and $R^{2a}$ are both halogen have activity against coccidial infections. Specifically disclosed are the compounds wherein $R^1$ is hydrogen, $R^{1a}$ is methyl, $R^{2a}$ is chlorine and $R^2$ is fluorine or chlorine and the corresponding compounds wherein $R^1$ and $R^{1a}$ are both methyl.

U.S. Patent No. 3862189 discloses, inter alia, that compounds of the formula (I) wherein $R^1$ and $R^{1a}$ are hydrogen or $C_{1-6}$ alkyl and $R^2$ and $R^{2a}$ are both halogen have antianginal or bronchial dilator activity. However, no specific examples of such compounds are given although the compound wherein $NR^1R^{1a}$ is an amino group and the phenyl ring is di-substituted at the meta and para positions by chlorine is exemplified.

German Patent application no. 2264727 discloses that compounds of the formula (II):

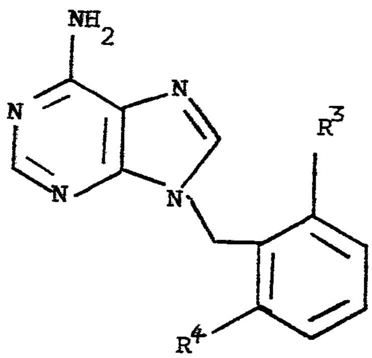

(II)

wherein $R^3$ and $R^4$ are halogen, have anti-coccidial activity. Compounds in which $R^3$ is chlorine and $R^4$ is chlorine or fluorine are specifically disclosed.

It has now been discovered that a group of novel 9- benzylpurines are active in the treatment of CNS disorders, such as psychiatric and neurological disorders, and are particularly useful as anticonvulsants, for example in the treatment of epilepsy. In addition, compounds of the present invention have antipsychotic activity.

However, the present compounds appear to have little or no activity in vivo in the treatment of coccidiosis.

The term "epilepsies" is a collective designation for a group of chronic central nervous system (CNS) disorders having in common the occurrence of sudden and transitory episodes (seizures) of abnormal phenomena of motor (convulsion), sensory, autonomic, or psychic origin. The seizures are nearly always correlated with abnormal and excessive EEG discharges.

As epileptic seizures in a susceptible patient occur without warning, preventive treatment can only be by way of continual administration of an effective drug. This puts a heavy demand on the quality of the drug, that is to say its freedom from, or relative freedom from, untoward side effects and neccessitates an absence of chronic toxicity.

Of the many drugs which are used in medicine for the treatment of epilepsies (see for example the work edited by Julius A. Vida "Anticonvulsants", Academic Press, 1977, and Goodman and Gilman's "The Pharmacological Basis of Therapeutics", 6th Edition, pages 448 to 474, both these works being incorporated herein by reference)

the compound 5,5-diphenylhydantoin (phenytoin), which was introduced for this purpose into medicine in 1938 has remained the most frequently used drug, in spite of its many disadvantages.

Accordingly, the present invention provides a compound of formula (III):

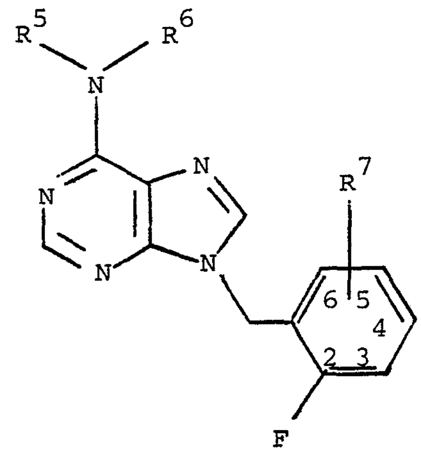

(III)

or an acid addition salt thereof, wherein $R^5$ is hydrogen, methyl or ethyl; $R^6$ is hydrogen, methyl, hydroxymethyl, methoxymethyl, ethyl, n-propyl, isopropyl, cyclopropyl, cyclopentyl, or cyclopropylmethyl and $R^7$ is hydrogen or fluorine attached to the 6- or 5- position of the phenyl ring.

Suitably the group $NR^5R^6$ is chosen from amino, methylamino, ethylamino, dimethylamino, diethylamino, propylamino, isopropylamino, cyclopropylamino, cyclopropylmethylamino, N-methylcyclopropylamino, N-methylethylamino, cyclopentylamino, hydroxymethylamino, methoxymethylamino and N-methoxymethyl-N-methylamino.

In a preferred aspect of the present invention, there is provided a compound of the formula (IV):

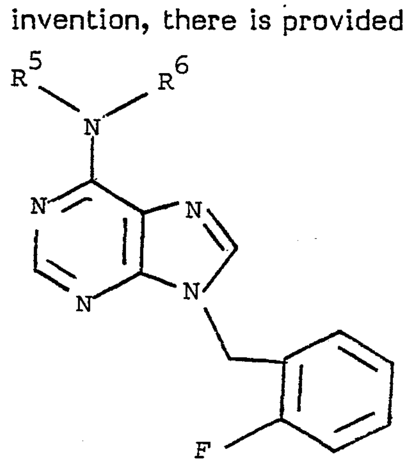

(IV)

or an acid addition salt thereof, wherein $R^5$ and $R^6$ are as hereinbefore defined.

Particularly preferred compounds of the formula (IV) are those wherein $R^5$ and $R^6$ are both hydrogen or wherein $R^5$ is hydrogen and $R^6$ is methyl.

Preferred compounds of the present invention include:

9-(2-Fluorobenzyl)-6-methylamino-9H-purine,
6-dimethylamino-9-(2-fluorobenzyl)-9H-purine,
6-cyclopropylamino-9-(2-fluorobenzyl)-9H-purine,
6-ethylamino-9-(2-fluorobenzyl)-9H-purine,
9-(2-fluorobenzyl)-6-isopropylamino-9H-purine,
6-cyclopropylmethylamino-9-(2-fluorobenzyl)-9H-purine,
9-(2-fluorobenzyl)-6-(N-methylcyclopropylamino)-9H-purine,
9-(2-fluorobenzyl-6-(N-methylethylamino)-9H-purine,
9-(2-fluorobenzyl)-6-propylamino-9H-purine,
6-diethylamino-9-(2-fluorobenzyl)-9H-purine,
6-cyclopentylamino-9-(2-fluorobenzyl)-9H-purine,
6-cyclopropylamino-9-(2,6-difluorobenzyl)-9H-purine,
9-(2,6-difluorobenzyl)-6-dimethylamino-9H-purine,
9-(2,5-difluorobenzyl)-6-methylamino-9H-purine,
6-amino-9-(2-fluorobenzyl)-9H-purine,
6-amino-9-(2,6-difluorobenzyl)-9H-purine,
6-amino-9-(2,5-difluorobenzyl)-9H-purine,
9-(2-fluorobenzyl)-6-hydroxymethylamino-9H-purine,
9-(2-fluorobenzyl)-6-(N-methoxymethyl-N-methylamino)-9H-purine,
9-(2-fluorobenzyl)-6-methoxymethylamino-9H-purine,

and acid addition salts thereof.

Suitable acid addition salts of the compounds of formula (III) include those formed with both organic and inorganic acids. Such acid addition salts will normally be pharmaceutically acceptable.

Thus, preferred salts include those formed from hydrochloric, sulphuric, citric, isethionic, tartaric, phosphoric, lactic, pyruvic, acetic, succinic, oxalic, fumaric, maleic, lactobionic, oxaloacetic, methanesulphonic, p-toluenesulphonic and benzenesulphonic acids.

The present invention also provides the first medical use of a compound of the formula (III), or a pharmaceutically acceptable salt thereof, as hereinbefore

defined. Preferably this will be for the treatment of CNS disorders, and in particular epilepsy, in humans. The compounds of the present invention have phenytoin like activity and are particularly useful in the treatment of grand mal epilepsy.

In a further aspect, the present invention provides pharmaceutical formulations comprising a compound of the formula (III) in admixture with a pharmaceutically acceptable carrier.

The pharmaceutically acceptable carriers present in the compositions of this invention are materials recommended for the purpose of administering the medicament. These may be liquid or solid materials, which are otherwise inert or medically acceptable and are compatible with the active ingredients.

These pharmaceutical compositions may be given orally or parenterally, used as a suppository, or applied topically as an ointment, cream or powder. However, oral and parenteral administration of the compositions are preferred.

For oral administration, fine powders or granules will contain diluting, dispersing and/or surface active agents, and may be presented in a draught, in water or in a syrup, in capsules or sachets in the dry state or in non-aqueous suspension wherein suspending agents may be included, or in a suspension in water or syrup. Where desirable or necessary, flavouring, preserving, suspending, thickening or emulsifying agents can be included.

For parenteral administration, the compounds may be presented in sterile aqueous injection solutions which may contain anti-oxidants or buffers.

As stated above, the free base or a salt thereof may be administered in its pure form unassociated with other derivatives, in which case a capsule or sachet is the preferred carrier.

Alternatively the active compound may be presented in a pure form as an effective unit dosage, for instance compressed as a tablet or the like.

Other compounds which may be included are, for example, medically inert ingredients, e.g. solid and liquid diluents such as lactose, starch or calcium phosphate for tablet or capsule; olive oil or ethyl oleate for soft capsules; and water or vegetable oil for suspensions or emulsions; lubricating agents such as talc

or magnesium stearate; gelling agents such as colloidal clays; thickening agents such as gum tragacanth or sodium alginate; and other therapeutically acceptable accessory ingredients such as humectants, preservatives, buffers, and antioxidants which are useful as carriers in such formulations.

Tablets or other forms of presentation provided in discrete units may conveniently contain an amount of compound of the formula (III) which is effective at such dosage or a multiple of the same, for instance, units containing 5mg to 500mg, usually around 10mg to 250mg.

The pharmaceutical compositions of the present invention will be prepared by the admixture of a compound of the formula (III) with a pharmaceutically acceptable carrier. Conventional pharmaceutical excipients may be admixed as required.

The present invention provides a method of treatment of CNS disorders, particularly epilepsy, in mammals, by the administration of a non-toxic therapeutically effective amount of a compound of the formula (III) or a pharmaceutically acceptable salt, or a composition as hereinbefore defined.

Preferably the mammal is a human.

Before commencement of the treatment the mammal in question will, in general, have been identified as suffering from a CNS disorder, particularly epilepsy.

Thus in a preferred embodiment of the present invention, there is provided a method of treatment of epilepsy in humans, comprising the adminstration to a human in need thereof of a non-toxic therapeutically effective amount of a compound of the formula (III) or a pharmaceutically acceptable salt, or a composition as hereinbefore defined.

As indicated above, the compounds of the formula (III) are generally useful in treating such disorders by administration to the human or animal recipient by a route selected from oral, parenteral (including subcutaneous, intradermal, intramuscular and intravenous) and rectal. The size of an effective dose of a compound will depend upon a number of factors including the mammal under treatment (for example cat, dog or human), the type of epilepsy involved for example grand mal, focal seizures and psychomotor convulsions, the severity of the condition to be treated and the route of administration, and will ultimately be at the discretion of the attendant physician. In guiding him in assessing the efficacy and acceptability of a regimen the physician will have recourse to changes in the

recipient's gross condition as treatment progresses.

Such an effective dose will generally be in the range 0.3 to 15 mg/kg bodyweight of animal or human recipient given three times per day, preferably in the range 0.5 to 7 mg/kg bodyweight and most preferably in the range of 1 to 2 mg/kg bodyweight. For the average human of 70 kg body weight at 1.0 mg/kg the dose would be 70 mg. Unless otherwise indicated all weights are calculated as the hydrochloride of formula (III). For other salts the figures would be amended proportionately. The desired dose may be preferably presented as between two and four sub-doses administered at appropriate intervals throughout the day.

The present invention also provides a process for the preparation of a compound of formula (III), which process comprises:

1) The reaction of an amine $HNR^5R^6$ with a compound of the formula (V):

(V)

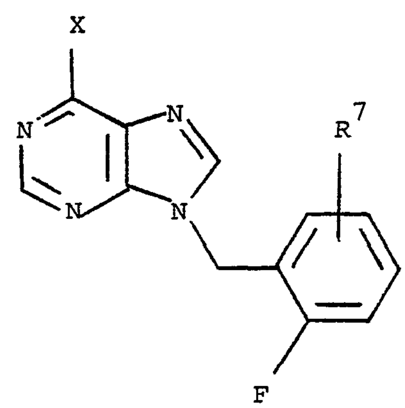

wherein $R^5$, $R^6$ and $R^7$ are as hereinbefore defined and X is a leaving group;

2) The reaction of a compound of the formula (VI) with a compound of the formula (VII):

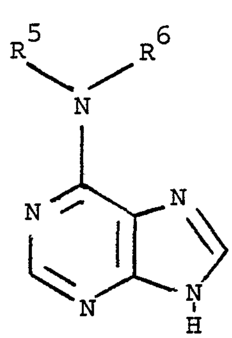

(VI)

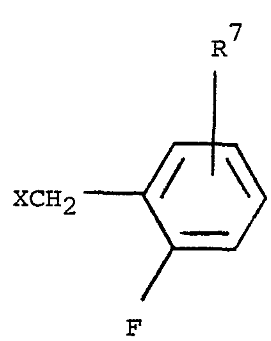

(VII)

wherein $R^5$, $R^6$, $R^7$ and X are as hereinbefore defined

3) The reduction of a compound of the formula (VIII):

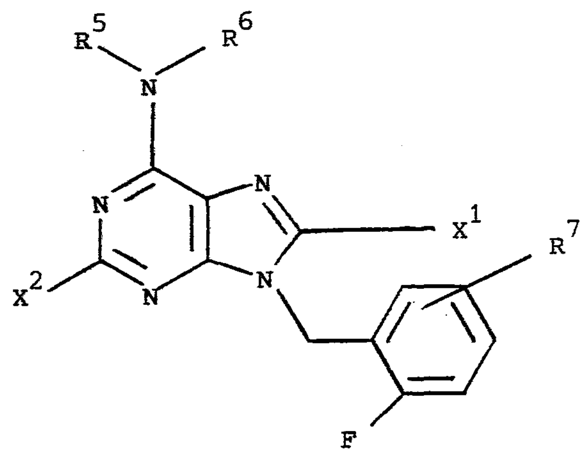

(VIII)

wherein $R^5$, $R^6$ and $R^7$ are as hereinbefore defined and $X^1$ and $X^2$ are the same or different and each is hydrogen, halogen, $C_{1-4}$ alkylthio or an aralkylthio group provided that at least one of $X^1$ and $X^2$ is other than hydrogen.

4) When it is required to form a compound of the formula (III) wherein $R^5$ is hydrogen, methyl or ethyl and $R^6$ is hydrogen, methyl, ethyl, propyl or cyclopropylmethyl, such that at least one of $R^5$ and $R^6$ is other than hydrogen, the reduction of a compound of the formula (IX):

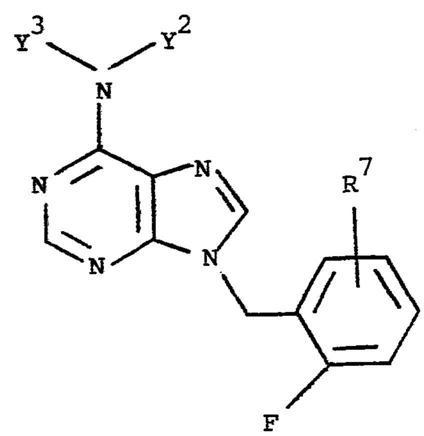

(IX)

wherein $R^7$ is as hereinbefore defined; $Y^2$ is a group $R^6$, $-(CH_2)n\ SR^8$ or $COR^9$ wherein $R^8$ is $C_{6-10}$ aryl, $C_{1-4}$ alkyl or $C_{1-10}$ arylalkyl, n = 1-3 and $R^9$ is hydrogen, cyclopropyl, methyl or ethyl and $Y^3$ is a group $R^5$, $-(CH_2)m\ SR^{8a}$ or $-COR^{10}$ wherein m is 1 or 2, $R^{8a}$ is $C_{1-4}$ alkyl and $R^{10}$ is hydrogen or methyl provided that $Y^2$ is not $R^6$ when $Y^3$ is a group $R^5$.

5) When it is required to form a compound of the formula (III) wherein $R^5$ is hydrogen and $R^6$ is methyl, ethyl or propyl, the base catalysed rearrangement of a compound of the formula (X) or its tautomeric salt (XI):

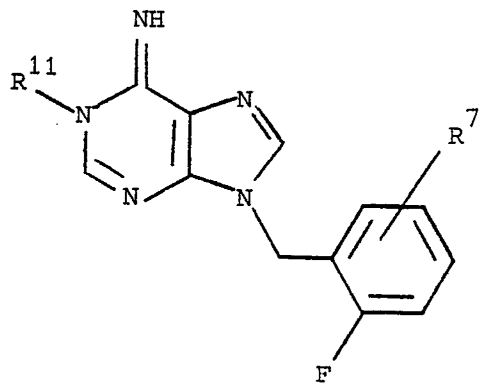

(X)

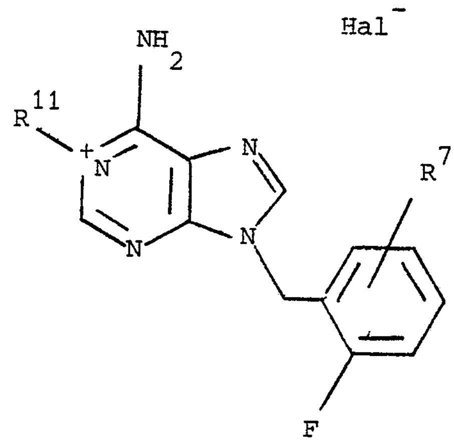

(XI)

wherein $R^{11}$ may be methyl, ethyl or propyl and $R^7$ is as hereinbefore defined.

6) When it is required to form a compound of the formula (III) wherein $R^5$ is hydrogen, (i) the base catalysed hydrolysis of a compound of the formula (XII);

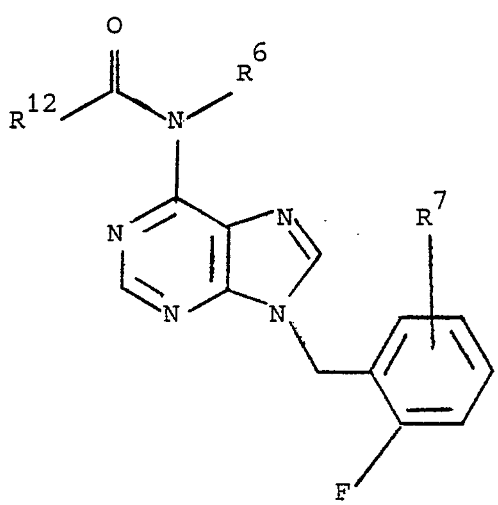

(XII)

wherein $R^6$ and $R^7$ are as hereinbefore defined and $R^{12}$ is hydrogen, $C_{1-10}$ alkyl, $C_{6-10}$ aryl or $C_{1-14}$ arylalkyl

(ii) The reduction of a compound of the formula (XIII):

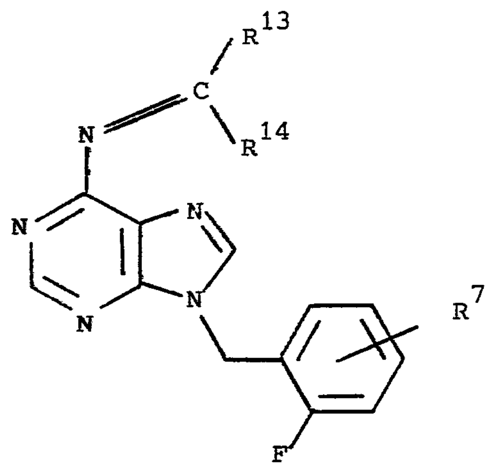

(XIII)

wherein $R^7$ is as hereinbefore defined and $R^{13}$ is a $C_{1-4}$ dialkylamino group and $R^{14}$ is hydrogen or a methyl, ethyl or cyclopropyl group.

7). The optional conversion of one compound of the formula (III) to another compound of the formula (III).

1) The reaction of a compound of the formula (V) with a monosubstituted or disubstituted amine will take place in any suitable solvent; preferably this will be a polar solvent such as a $C_{1-4}$ alkanol, water or acetonitrile. Where appropriate the amine may be used as a co-solvent.

The reaction will be carried out at non extreme temperatures eg. 0-180$^oC$, suitably at 15 - 120$^oC$ and conveniently at room temperature.

Suitable leaving groups X include halogen, $C_{1-6}$ alkylthio, $C_{1-10}$ arylthio, $C_{7-12}$ aralkylthio or $C_{1-4}$ alkyl, phenyl, benzyl, phenethyl or naphthylmethyl, substituted sulphonyl or sulphinyl. Preferred leaving groups include halogen such as chlorine, $C_{1-6}$ alkylthio, $C_{6-10}$ arylthio, $C_{7-12}$ aralkylthio or substituted sulphonyl.

Compounds of the formula (V) may be synthesised by treating a compound of the formula (XIV):

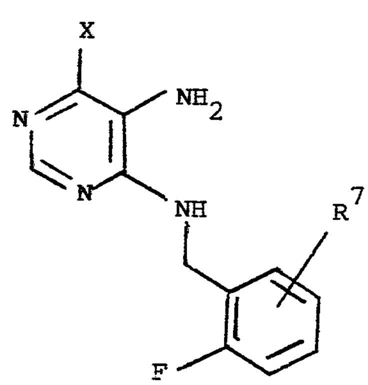

(XIV)

where X and $R^7$ are as hereinbefore defined with an $C_{1-4}$ alkylorthoformate, e.g in the presence of a suitable acid catalyst such as ethanesulphonic acid under conditions similar to those described in US patent 3,936,454, or may be prepared from a compound of the formula (XV):

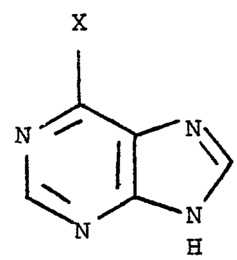

(XV)

according to methods well known to those skilled in the art, for example J. Amer. Chem. Soc., 83, 633 (1961). Compounds of the formula (XIV) suitably may be prepared by the reaction of an appropriate substituted benzylamine with 5-amino-4,6-dichloropyrimidine under the conditions described in U.S. Patent No. 3,862, 189.

2. The reaction of compounds of the formula (VI) with compounds of the formula (VII) may suitably be accomplished in dipolar aprotic solvents such as dimethylformamide or dimethylsulphoxide in the presence of a base such as potassium carbonate, sodium hydride, sodium hydroxide or metal alkoxides, as described in US Patent No. 3,936,454. The reaction will take place at temperatures between $25^o$ and $180^oC$, suitably in the range 50 - $150^oC$ and conveniently at 50-$100^oC$. The leaving group X is suitably halogen or a $C_{1-4}$ alkyl or $C_{7-12}$ aralkyl sulphonate group eg. mesyloxy or tosyloxy.

Compounds of the formula (VI) may be synthesised according to methods described in Fused Pyrimidines Part II by J.H. Lister, Wiley Interscience 1971 Chapter 8.

3) The reduction of a compound of the formula (VIII) suitably may be achieved by treatment with Raney nickel in an alkanol solution at elevated temperatures eg. 50-$100^oC$ or by hydrogenolysis over a

transition metal catalyst such as palladium or Raney nickel. The catalytic hydrogenation suitably is carried out in a polar solvent such as an alkanol, optionally in the presence of an alkanoate salt such as sodium acetate, or is carried out in an organic or mineral acid, preferably in aqueous or glacial acetic acid or hydrochloric acid, at a non-extreme temperature, suitably in the range 0-100$^{o}$C but conveniently at room temperature.

Compounds of the formula (VIII) suitably are prepared by reaction of a compound of the formula (VIIIA):

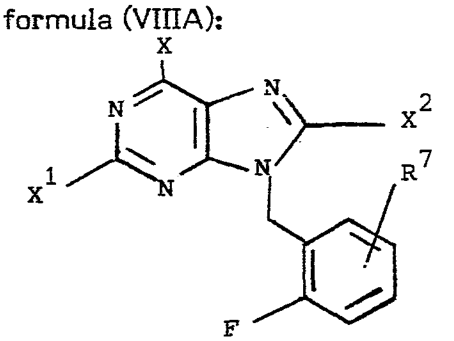

(VIIIA)

wherein X, $X^1$, $X^2$ are $R^7$ are as hereinbefore defined, with an approriately substituted amine under conditions similar to those described in respect of process variant 1) above. Compounds of the formula (VIIIA) suitably are prepared from compounds of the formula (VIIIB);

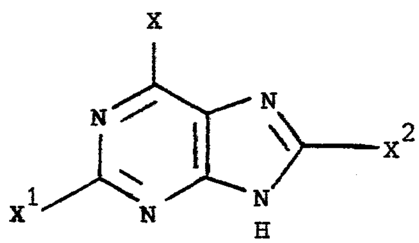

(VIIIB)

under conditions analogous to those described in respect of process variant 2) as hereinbefore defined.

4) The reduction of a compound of the formula (IX), wherein $Y^2$ and/or $Y^3$ are groups $-COR^9$ and/or $-COR^{10}$ respectively, suitably takes place in the presence of a hydride reducing agent eg. lithium aluminium hydride. Such a reaction conveniently takes place at elevated temperatures in an inert solvent such as tetrahydrofuran, diethylether or dioxan. When $Y^3$ and/or $Y^2$ are $-(CH_2)n\ SR^8$, the reduction suitably may be accomplished by reaction with Raney nickel in a polar solvent at elevated temperatures or, when n=1, by reaction with a complex hydride such as sodium borohydride in an inert solvent such as a glycol ether.

Compounds of the formula (IX) wherein $Y^2$ and/or $Y^3$ are groups $COR^9$ and/or $COR^{10}$ respectively can be prepared by acylation of the appropriate compound of the formula (III), wherein one or both of $R^5$ and $R^6$ is or are hydrogen, according to techniques well known in the art. Thus, for example acylation may be effected by using the appropriate carboxylic acid or an activated derivative thereof such as the anhydride or a mixed anhydride.

Compounds of the formula (IX) wherein $Y^2$ and/or $Y^3$ are $-CH_2SR^8$ and/or $-CH_2SR^{8a}$ respectively suitably are prepared by reaction of a compound of the formula (III) wherein one or both of $R^5$ and $R^6$ is or are hydrogen with an alkyl-, aryl- or aralkylthiol in the presence of formaldehyde.

5) The rearrangement of compounds of the formula (X) or (XI) suitably takes place in aqueous, alkanolic or aqueous alkanolic solution, suitably in the presence of a base such as sodium hydroxide and in the temperature range 5-100$^oC$ conveniently 15-80$^oC$ and preferably room temperature. Such a rearrangement is known to those skilled in the art as a Dimroth rearrangement and is described in Fused Pyrimidines Part II. J.H. Lister, Wiley Interscience 1971, pp 313-16 and German Patent Application No. 2836373. Compounds of the formulae (X) or (XI) are conveniently prepared by reacting a compound of the formula (III), wherein $R^5$ and $R^6$ are both hydrogen, with an alkylating agent in a polar solvent. The reaction will be carried out normally at temperatures in the range 0-100$^oC$ and conveniently at ambient temperature. The alkylating agent suitably is an alkyl halide, alkyl benzensulphonate or tosylate, or an alkyl sulphate and coveniently is an alkyl halide. The solvent suitably is a polar aprotic solvent such as dimethylformamide or dimethylacetamide.

6) i) Hydrolysis of amides of the formula (XII) suitably may be performed by reacting them with aqueous base eg. sodium hydroxide or alkoxide or an amine suitably in the presence of an organic co-solvent and at a temperature in the range 0-150$^oC$, preferably 20-100$^oC$. Amides of the formula (XII) suitably may be prepared from compounds of the formula (III) by methods well known to those skilled in the art, for example by oxidation of a compound of the formula (III) wherein $R^5$ is methyl.

ii) The reduction of a compound of the formula (XIII) suitably may be achieved by reaction with sodium borohydride, or another metal hydride

reagent, in a polar solvent such as an alkanol, or a dipolar aprotic solvent such as dimethylsulphoxide at temperatures suitably in the range 50°-200°C but conveniently at 100 to 150°C (see for example US Patent 4361699). Amidines of the formula (XIII) suitably may be prepared from compounds of the formula (III) ($R^5=R^6=H$) under conditions well known to those skilled in the art e.g. see European Patent Application No. 82305072.9.

Optional conversions of one compound of the formula (III) into another compound of the formula (III) include, for example, transformation into a compound of the formula (IX) and subsequent reduction as described hereinbefore in process variant (4); or alkylation to give a compound of the formula (X) or (XI) followed by Dimroth rearrangement as described in process variant (5). When it is desired to remove an alkyl group from the 6-amino function, this is suitably achieved by oxidation of an N-alkyl group to an N-acyl group to give an amide of the formula (XII) followed by hydrolysis as described hereinbefore in process variant (6(i)).

When it is desired to N-alkylate a compound of the formula (III) wherein $R^5$ and $R^6$ are both hydrogen, this can be achieved for example by reacting the amine with the acetal of an amide such as dimethylformamide dimethylacetal to give a compound of the formula (XIII), followed by reduction as described in process variant (6(ii)).

Alternatively the compound of the formula (III) wherein $R^5$ and $R^6$ are hydrogen may be reductively alkylated according to known methods, or subjected to an activation/alkylation/hydrolysis sequence of reactions. Typically such a sequence comprises formation of an amide or sulphonamide, for example by reaction with an appropriate derivative of acetic acid or methane-, ethane- toluenesulphonic acids, followed by alkylation in the presence of a base such as sodium or potassium carbonate or sodium hydride, and subsequent hydrolysis of the acyl derivative to yield a compound of the formula (III) bearing a monosubstituted amino group in the 6-position.

Further optional interconversions include hydroxymethylation of the 6-amino group, for example by treatment of a compound of the formula (III) wherein $R^6$ is hydrogen with formaldehyde, preferably at room temperature. The corresponding methoxymethyl-derivative can in turn be prepared by reaction of the hydroxymethyl- compound with methanol in strong acid, eg. concentrated hydrochloric acid. Alternatively the methoxymethyl derivative can be prepared by heating the appropriate unsubstituted or monosubstituted amino-group with

formaldehyde. This reaction suitably is carried out at approximately 50°C and in the presence of a base such as dilute sodium hydroxide.

Novel intermediates of the formulae (V) and (VIII) - (XIII) form a further aspect of the present invention.

The following examples illustrate the invention. These examples are not intended to limit the invention.

Example 1: 9-(2-Fluorobenzyl)-6-methylamino-9H-purine

(A) 5-Amino-4-chloro-6-(2-fluorobenzylamino)pyrimidine
A mixture of 5-amino-4,6-dichloropyrimidine (12.6 g, 76.8 mmol), 2-fluorobenzylamine (96%) (10.0 g, 76.7 mmol), 1-butanol (150 ml) and triethylamine (8.0 g 79.2 mmol) was refluxed with stirring for 24 hr. The dark solution was cooled to give a solid that was collected on a Buchner funnel and washed with cyclohexane. The white solids were dispersed in water (50 ml), collected, and dried to give 15.97g (82% of theory) of 5-amino-4-chloro-6-(2-fluorobenzylamino)pyrimidine, mp 218-221°C. Recrystallization of a portion from ethanol gave the analytical sample, mp 220-223°C.

Elemental Analysis:

| | |
|---|---|
| Calcd for $C_{11}H_{10}ClFN_4$ (MW 252.68): | C, 52.28; H, 3.99; N, 22.17. |
| Found: | C, 52.39; H, 3.99; N, 22.07. |
| TLC: | ethyl acetate-cyclohexane (1:2) |

(B) 6-Chloro-9-(2-fluorobenzyl)-9*H*-purine
A mixture of 5-amino-4-chloro-6-(2-fluorobenzylamino)pyrimidine (12.55 g, 49.7 mmol), triethylorthoformate (100 ml) and ethanesulfonic acid (90 mg) was stirred at ambient temperature for 41 hr. The solution was spin evaporated *in vacuo*. The residual solid was dissolved in ethyl acetate (200 ml) and washed sequentially with 5% aqueous sodium bicarbonate (50 ml), water (4 x 50 ml), brine (50 ml) and then dried with magnesium sulfate. The solution was spin evaporated *in vacuo* to a residue that was collected and washed with cyclohexane to give 12.41 g (95% of theory) of 6-chloro-9-(2-fluorobenzyl)-9*H*-purine, mp 96-98°C. Recrystallization from cyclohexane-ethyl acetate gave the analytical sample, mp 97-99°C.

Elemental Analysis: Calcd for $C_{12}H_8ClFN_4$ (MW 262.67): C, 54.87; H, 3.07; N, 21.33.
Found:C, 54.96; H, 3.04; N, 21.15.
TLC: ethyl acetate-cyclohexane (1:2)

(C)9-(2-Fluorobenzyl)-6-methylamino-9H-purine

A solution of 6-chloro-9-(2-fluorobenzyl)-9H-purine (5.00 g, 19.0 mmol), ethanol (40 ml) and 40% aqueous monomethylamine (10 ml) was stirred at ambient temperature for 15 hr. The reaction mixture was spin evaporated to remove the volatiles. The residue was dispersed in water (70 ml) and the solids were collected. Recrystallization from ethyl acetate (Norite) gave 2.86 g (58% of theory) of 9-(2-fluorobenzyl)-6-methylamino-9H-purine, mp 151-153°C.

| | |
|---|---|
| Elemental Analysis: | Calcd for $C_{13}H_{12}FN_5$ (MW 257.27): C, 60.69; H, 4.70; N, 27.22. |
| Found: | C, 60.83; H, 4.67; N, 27.25. |
| TLC: | ethyl acetate |

D) 9-(2-Fluorobenzyl)-6-methylamino-9H-purine hydrochloride
To a solution of 9-(2-fluorobenzyl)-6-methylamino-9H-purine (36.0 g, 140 mmol) in warm ethanol (500 ml) was added 12 M hydrochloric acid (15 ml) in ethanol (50 ml). The resultant mixture was spin evaporated *in vacuo* to remove the volatiles. The white residue was recrystallized from ethanol-water to give 37.11 g (90% of theory) of 9-(2-fluorobenzyl)-6-methylamino-9H-purine hydrochloride, mp 255-259°C (prior wetting at approximately 245°C).

| | |
|---|---|
| Elemental Analysis: | Calcd for $C_{13}H_{12}FN_5HCl$ (MW 293.735): C, 53.15; H, 4.46; N, 23.8 |
| Found: | C, 53.17; H, 4.51; N, 23.83. |
| TLC: | ethyl acetate-ethanol (20:3) |

## EXAMPLES 2 to 14

The compounds of Examples 2 to 14 were prepared by a method exactly analogous to that employed in Example 1, using appropriately substituted starting materials.

| Example | Compound | MP, °C |
| --- | --- | --- |
| 2. | 6-Dimethylamino-9-(2-fluorobenzyl)-9H-purine | 137-139 |
| 3. | 6-Cyclopropylamino-9-(2-fluorobenzyl)-9H-purine | 133-134 |
| 4. | 6-Ethylamino-9-(2-fluorobenzyl)-9H-purine | 143-143.5 |
| 5. | 9-(2-fluorobenzyl)-6-isopropylamino-9H-purine | 98-100 |
| 6. | 6-(Cyclopropylmethylamino)-9-(2-fluorobenzyl)-9H-purine hydrochloride | 199-201 |
| 7. | 9-(2-Fluorobenzyl)-6-(N-methylcyclopropylamino)-9H-purine | 100-103 |
| 8. | 9-(2-Fluorobenzyl)-6-(N-methylethylamino)-9H-purine hydrochloride | 153-156 |
| 9. | 9-(2-Fluorobenzyl)-6-propylamino-9H-purine hydrochloride | 200-204 |
| 10. | 6-Diethylamino-9-(2-fluorobenzyl)-9H-purine hydrochloride | 168-171 |
| 11. | 6-Cyclopentylamino-9-(2-fluorobenzyl)-9H-purine hydrochloride | 160-163 |
| 12. | 6-Cyclopropylamino-9-(2,6-difluorobenzyl)-9H-purine hydrochloride | 237-240 |
| 13. | 9-(2,6-Difluorobenzyl)-6-methylamino-9H-purine hydrochloride | 282-285 |
| 14. | 9-(2,6-Difluorobenzyl)-6-dimethylamino-9H-purine hydrochloride | 220-225 |

EXAMPLE 15: 9-(2,5-Difluorobenzyl)-6-methylamino-9H-purine

(A) 6-Chloro-9-(2,5-difluorobenzyl)-9H-purine

A mixture of 6-chloropurine[1] (5.00 g, 32.3 mmol), dimethylsulfoxide (75 ml), anhydrous potassium carbonate (5.00 g, 36.2 mmol), and 2,5-difluorobenzyl bromide (which was prepared in a conventional way[3] from 2,5-difluorotoluene and N-bromosuccinimide) (6.47 g, 31.2 mmol) was stirred at ambient temperature for 39 hr. The reaction solution was decanted from the solids, poured into ice water and acidified to pH 5 with acetic acid (0.5 ml). The mixture was extracted with dichloromethane (4 x 100 ml) and the combined extracts were washed with water (5 x 50 ml), dried (magnesium sulfate) and spin evaporated in vacuo. The residue was dissolved in dichloromethane (50 ml) and added to silica gel 60 (50 g) (40-63 µm, 230-400 mesh, E. Merck No. 9385). This mixture was spin evaporated in vacuo and the residual solids were introduced onto a column of silica gel 60(40-63) µm, 230-400 mesh, E. Merck No. 9385) wetted with ethyl acetate:cyclohexane/1:1. The column was eluted with ethyl acetate:cyclohexane/1:2 using the "flash chromatography technique".[2] The fractions containing the higher $R_f$ major component were combined and spin evaporated in vacuo to give 3.50 g (40% of theory) of 6-Chloro-9-(2,5-difluorobenzyl)-9H-purine which showed only a single spot by TLC analysis.

[1] J.A. Montgomery, J.Amer.Chem.Soc., 78, 1928 (1956).

[2] W.C. Still, M. Kahn and A. Mitra, J. Org. Chem., 43, 2923 (1978).

[5] C. Djerassi. Chem.Rev., 43, 271 (1948)

(B) 9-(2,5-Difluorobenzyl)-6-methylamino-9H-purine hydrochloride

A solution of 6-chloro-9-(2,5-difluorobenzyl)-9H-purine (3.50 g, 12.5 mmol), ethanol (50 ml) and 40% aqueous monomethylamine (20 ml) was stirred at ambient temperature for 15 hr. The reaction mixture was spin evaporated to remove the volatiles. The residue was dispersed in $H_2O$ (70 ml) and the solids were collected. To this product dissolved in warm ethanol (50 ml) was added 12 M hydrochloric acid (15 ml) in ethanol (5 ml). The resultant mixture was spin evaporated in vacuo to remove the volatiles. The white residue was recrystallized from ethanol-water to give 2.62 (67% of theory) of 9-(2,5-difluorobenzyl)-6-methylamino-9H-purine hydrochloride, mp 265-268°C.

| | |
|---|---|
| Elemental Analysis: | Calcd for $C_{13}H_{11}F_2N_5$ HCl (MW 311.727): C, 50.08; H, 3.88; N, 22.4 |
| Found: | C,50.17; H, 3.90; N, 22.43. |
| TLC: | ethyl acetate-ethanol (20:3) |

EXAMPLE 16: 6-Amino-9-(2-fluorobenzyl)-9H-purine

To a solution of 6-cyclopropylamino-9-(2-fluorobenzyl)-9H-purine (5.00 g, 17.6 mmol), 0.5 N sodium acetate pH 4 buffer (100 ml), and tetrahydrofuran (100 ml) was added bromine (2 ml). After 10 minutes an additional portion of bromine (2 ml) was added. The reaction was stirred for 20 minutes and a slurry of sodium sulfite (12 g) in water was added to reduce the excess bromine. The solution was extracted with ethyl acetate (3 x 400 ml), the combined extracts were washed with brine, dried (magnesium sulfate), and spin evaporated in vacuo. The residue was combined with the product from a separate reaction on 6-cyclopropylamino-9-(2-fluorobenzyl)-9H-purine (6.20 g, 21.9 mmol) and dissolved in methanol. This solution was added to silica gel 60 (20 g) (40-63 µm, 230-400 mesh, E. Merck No. 9385) and spin evaporated in vacuo. The residual solids were introduced on a column (20 cm x 40 cm) of silica gel 60 (40-63 µm 230-400 mesh, E. Merck No. 9385) wetted with 1% methanol in chloroform. The column was successively eluted with 1% methanol in dichloromethane (2 liters), 2% methanol in dichloromethane (1 liter) and finally with 3% methanol in dichloromethane using the "flash chromatography technique".[2] The fractions containing product were combined, spin evaporated in vacuo and recrystallized from ethanol to give 5.00 g (52% of theory) of 6-amino-9-(2-fluorobenzyl)-9H-purine, mp 237.5-238.50.

| | | |
|---|---|---|
| Elemental Analysis: | Calcd for $C_{12}H_{10}FN_5$ (MW 243.25): | C, 59.25; H, 4.14; N, 28.79. |
| Found: | C, 59.11; H, 4.01; N, 28.78. | |
| TLC: | methanol:dichloromethane (1:9). | |

Example 17

Pharmacological Activity

(i) Anticonvulsant Activity in rats.

The anticonvulsant activity of certain compounds of the present invention was determined by a standard maximal electroshock test (MES); that described by L.A. Woodbury and V.D. Davenport, Arch. Int. Pharmacodyn., 1952, 92, 97.

Compounds of the formula (III) MES $ED_{50}$ (mg/kg)

| | $R^5$ | $R^6$ | $R^7$ | i.p. | p.o. |
|---|---|---|---|---|---|
| 1 | H | $CH_3$ | H | 1.7±0.4 | 2.5±0.4 |
| 2 | $CH_3$ | $CH_3$ | H | 8.8±1.7 | - |
| 3 | H | cyclopropyl | H | 5.0±0.4 | 7.8±0.8 |
| 4 | H | $C_2H_5$ | H | 4.9±0.5 | - |
| 5 | H | $CH(CH_3)_2$ | H | 5.8±0.6 | - |
| 6 | H | cyclopropylmethyl | H | 4.3±0.9 | 6.3±1.0 |
| 7 | $CH_3$ | cyclopropyl | H | 6.8±0.8 | - |
| 8 | $CH_3$ | $C_2H_5$ | H | 2.9±0.3 | 6.0±0.5 |
| 9 | H | $C_3H_7$ | H | 5.1±0.5 | 4.6±1.0 |
| 10 | $C_2H_5$ | $C_2H_5$ | H | 4.2±0.5 | 5.9±0.6 |
| 11 | H | cyclopentyl | H | 8.0±1.0 | - |
| 12 | H | cyclopropyl | 6-F | 4.0 | 12.5 |
| 13 | H | $CH_3$ | 6-F | 2.0 | - |
| 14 | $CH_3$ | $CH_3$ | 6-F | 2.2±0.2 | 3.8±0.6 |
| 15 | H | $CH_3$ | 5-F | 4.0±0.6 | 7.0±1.0 |
| 16 | H | H | H | 2.4±0.6 | 2.2±0.4 |
| 27 | H | H | 6-F | 9.0 | - |
| 28 | H | H | 5-F | 5.0 | - |
| 29 | H | $CH_2OH$ | H | 4.0 | - |
| 30 | $CH_3$ | $CH_2OCH_3$ | H | 5.0 | - |
| 31 | H | $CH_2OCH_3$ | H | (17% at 6.25mg/kg) | |
| phenytoin (Dilantin) | | | | 10±2 | 20±3 |

(ii) Anticoccidial Activity

The *in vivo* activity of two compounds of the present invention and a compound disclosed in US Patent No. 4189485 were determined against *Eimeria tenella* by the method disclosed in European Patent No. 0021293.

| | $R^5$ | $R^6$ | $R^7$ | Concentration (ppm) | E.tenella cleared |
|---|---|---|---|---|---|
| 1 | H | $CH_3$ | F | 400 | 2/5 |
| 2 | $CH_3$ | $CH_3$ | F | 400 | 0/5 |
| | $-CH_3$ | $CH_3$ | Cl | 400 | 5/5 |

(iii) $LD_{50}$ - Compound 1 has an approximate $LD_{50}$ of 1000 mg/kg (p.o.) in the rat.

EXAMPLE 18: 6-Amino-9-(2-fluorobenzyl)-9H-purine

A mixture of 6-chloro-9-(2-fluorobenzyl)-9H-purine (10.00 g, 38.07 mmol) and ammonia saturated ethanol (300 ml) in a glass lined stainless steel reaction vessel was heated (120°C) for 48 hr. The reaction mixture was cooled, and the solids were collected on a Buchner funnel. Recrystallization from ethanol gave 7.11 g (76% of theory) of 6-amino-9-(2-fluorobenzyl)-9H-purine, mp 243-245°C.

Elemental Analysis: Calcd for $C_{12}H_{10}FN_5$ (MW 243.25): C, 59.25; H, 4.14; N,28.79.
Found: C, 59.19; H, 4.15; N, 28.76.

TLC: ethyl acetate

EXAMPLE 19 9-(2-Fluorobenzyl)-6-methylamino-9H-purine

To a stirred dispersion of sodium hydride (60.2% dispersion in mineral oil) (0.129 g, 3.23 mmol) in dry dimethylsulfoxide (10 ml) was added 6-methylaminopurine[4] (0.442 g, 2.96 mmol). The reaction mixture was stirred at ambient temperature for 1 hr. 2-Fluorobenzyl bromide (98%) (0.575 g, 2.98 mmol) was added and the solution was stirred for 24 hr. The reaction was diluted with water (100 ml) and extracted with dichloromethane (4 x 25 ml). The combined extracts were washed with water (5 x 20 ml), filtered through glass wool and spin evaporated in vacuo. The residue was dissolved in dichloromethane (25 ml) and added to silica gel 60 (15 g) (40-63 μm, 230-400 mesh, E. Merck No. 9385). This mixture was spin evaporated in vacuo, and the residual solids were introduced onto a column of silica gel 60 wetted with ethyl acetate. The column was eluted with

ethyl acetate using the "flash chromatography technique"[2]. The fractions containing the product were combined and spin evaporated in vacuo to give 0.340 g (44% of theory) of product, mp 145-148°C. These solids were dispersed in a few ml. of ethyl acetate and collected to give 0.170 g (22% of theory) of 9-(2-fluorobenzyl)-6-methylamino-9H-purine, mp 150-152°C which was identical to that prepared in example 1.

[4] G. B. Elion, E. Burgi and G.H. Hitchings, J. Am. Chem. Soc., 74 411 (1952).

EXAMPLE 20: 9-(2-Fluorobenzyl)-6-methylamino-9H-purine

(A) 6,8-Dichloro-9-(2-fluorobenzyl)-9H-purine

A mixture of 6,8-dichloro-9H-purine[5] (3.0 g, 15.9 mmol), 2-fluorobenzyl bromide (3.0g, 15.9 mmol), anhydrous potassium carbonate (3.3 g, 23.9 mmol), and dimethylformamide (30 ml) was stirred for 18 hr at ambient temperature. The dark solution was diluted with water (400 ml) and extracted with ethyl acetate (6 x 100 ml). The ethyl acetate extracts were combined, dried with magnesium sulfate and added to silica gel 60 (40-63 µm, E. Merck No. 9385). This mixture was spin evaporated in vacuo and the residual solids were added to a column (5 cm x 20 cm) of silica gel 60 (40-63 µm, E. Merck No. 9385) wetted with ethyl acetate:hexane/1:1. The column was eluted with ethyl acetate:hexane/1:1 using the "flash chromatography technique"[2]. The appropriate fractions were combined, spin evaporated in vacuo, and the white residue was recrystallized from hexane to give 2.1g (45% of theory) of 6,8-dichloro-9-(2-fluorobenzyl)-9H-purine, mp 104-105°C.

Elemental Analysis: Calc'd for $C_{12}H_7Cl_2FN_4$ (MW 297.12): C 48.51; H, 2.37; N, 18.86.
Found: C, 48.53; H, 2.39; N, 18.86.

TLC: ethyl acetate:hexanes (1:2)

[5] R.K. Robins, J. Am. Chem. Soc., 80, 6671 (1958).

(B) 8-Chloro-9-(2-fluorobenzyl)-6-methylamino-9H-purine

A mixture of 6,8-dichloro-9-(2-fluorobenzyl)-9H-purine (1.50g, 5.04 mmol), 40% aqueous methylamine (5 ml), and ethanol (15 ml) was stirred in a sealed flask

for 2 hr at ambient temperature. The reaction mixture was combined with material from a similar reaction (1.5 mmol scale) and spin evaporated in vacuo. The residual solid was dissolved in ethyl acetate:methanol, added to silica gel 60 (40-63 µm, E. Merck No. 9385) and spin evaporated in vacuo. The dry solids were added to a column (5 cm x 25 cm) of silica gel 60 (40-63 µm, E. Merck No. 9385) wetted with ethyl acetate:hexane/1:3. The column was eluted with ethyl acetate:hexanes/1:3 using the "flash chromatography technique"[2]. The appropriate fractions were combined and spin evaporated in vacuo. Recrystallization of the white solid from ethyl acetate -hexanes gave 0.80 g (42% of theory) of 8-chloro-9-(2-fluorobenzyl)-6-methylamino-9H-purine, mp 181.5-182°C.

Elemental Analysis: Calc'd for $C_{13}H_{11}ClFN_5$ (MW 291.72); C, 53.53; H, 3.80; N, 24.01.

Found: 53.41; H, 3.84; N, 23.93.

TLC: Ethyl acetate

(C) 9-(-2-Fluorobenzyl)-6-methylamino-9H-purine

A mixture of 8-chloro-9-(2-fluorobenzyl)-6-methylamino-9H-purine (0.200g, 0.69 mmol), anhydrous sodium acetate (0.125g, 1.52 mmol), 5% palladium on carbon (30 mg) and ethanol (25 ml) was shaken in the presence of hydrogen at 2-3 atmospheres pressure for 4 hr at ambient temperature. The reaction mixture was filtered, and the filtrates were spin evaporated in vacuo. The white residue was dissolved in ethyl acetate (50 ml), washed with water (3 x 20 ml) and dried ($MgSO_4$). The ethyl acetate solution was spin evaporated, and the residue was recrystallized from ethyl acetate-hexanes to give 0.103 g (58% of theory) of 9-(2-fluorobenzyl)-6-methylamino-9H-purine, mp 152.5-154°C which was identical to that prepared in Example 1.

EXAMPLE 21: 9-(2-Fluorobenzyl)-6-methylamino-9H-purine

(A) 2,6-Bis-methylthio-9-(2-fluorobenzyl)-9H-purine

A mixture of 2,6-bis-methylthio-9H-purine[6] (3.20 g, 15.1 mmol), 2-fluorobenzyl bromide (3.14g, 16.6 mmol), anhydrous potassium carbonate (2.5g, 18.3 mmol) and dimethylformamide (20 ml) was stirred for 66 hr at ambient temperature. The reaction was diluted with water (100 ml) and extracted with ethyl acetate

(4 x 100 ml). The ethyl acetate extracts were combined and washed with water (25 ml), dried with magnesium sulfate and spin evaporated in vacuo. The residual oil was dissolved in ethyl acetate, added to silica gel 60 (40-63 µm, E. Merck No. 9385) and spin evaporated in vacuo. The residual solids were introduced onto a column of silica gel 60 (40-63 µm, E. Merck No. 9385) wetted with ethyl acetate:hexanes/1:4. The column was eluted using the "flash chromatography technique"[2].

Fractions containing the major component were combined and spin evaporated in vacuo. Recrystallization from ethyl acetate-hexane gave 2.20g (45% of theory) of 2,6-bis-methylthio-9-(2-fluorobenzyl)-9H-purine, mp 110.0-110.5°C.

Elemental Analysis: Calc'd for $C_{14}H_{13}FN_4S_2$ (MW 320.41): C, 52.48; H, 4.09, N, 17.49. Found:C, 52.54; H, 4.11; N, 17.49.

TLC: ethyl acetate-hexane (1:2)

[6] C. W. Noell and R.K. Robins J. Am. Chem. Soc., 81, 5997 (1959).

(B) 9-(2-Fluorobenzyl)-6-methylamino-2-methylthio-9H-purine

A solution of 2,6-bis-methylthio-9-(2-fluorobenzyl)-9H-purine (1.50g, 4.68 mmol), 40% aqueous methylamine (30 ml) and water (20 ml) was heated in a 200 ml stainless steel reaction vessel at 130°C for 4 hr. The cooled reaction mixture was spin evaporated in vacuo and the residual solids were recrystallized from ethyl acetate-cyclohexanes to give 0.84g (59% of theory) of 9-(2-fluorobenzyl)-6-methylamino-2-methylthio-9H-purine, mp 154-155°C.

Elemental Analysis: Calc'd for $C_{14}H_{15}FN_5S$ (MW 302.357): C, 55.61; H, 4.33; N, 23.16. Found:C, 55.18; H, 4.69; N, 22.83.

TLC: Methanol-dichloromethane (1:9)

(C) 9-(2-Fluorobenzyl)-6-methylamino-9H-purine

A mixture of 9-(2-fluorobenzyl)-6-methylamino-2-methylthio-9H-purine (0.100g, 0.31 mmol), Raney Nickel catalyst (4g), and ethanol (10 ml) was refluxed for 18 hr. The mixture was filtered, and the catalyst was washed with ethanol (75 ml) and water (75 ml). The filtrate and washes were combined and spin evaporated in vacuo. The white residue was recrystallized from ethyl

acetate-cyclohexane to give 0.033g (41% of theory) of 9-(2-fluorobenzyl)-6-methylamino-9H-purine, mp 153-154°C which was identical to that prepared in Example 1.

EXAMPLE 22: 9-(2-Fluorobenzyl)-6-methylamino-9H-purine

(A) 9-(2-Fluorobenzyl)-6-formamido)-9H-purine

To an ice cold solution of anhydrous formic acid (5 ml), acetic anhydride (10 ml) and dichloromethane (50 ml) was added in small portions 4-dimethylaminopyridine (0.75g, 6.17 mmol). The solution was allowed to come to ambient temperature while being stirred under a nitrogen atmosphere. To this stirred solution was added 6-amino-9-(2-fluorobenzyl)-9H-purine (1.50g, 6.17 mmol). After 66hr the reaction was diluted with dichloromethane (400 ml) and washed with water (50 ml), 5% sodium bicarbonate (50 ml), water (50 ml) and dried ($MgSO_4$). The solution was spin evaporated in vacuo and the residue was recrystallized from ethyl acetate-hexanes to give 1.50g (90% of theory) of 9-(2-fluorobenzyl)-6-formamido-9H-purine, mp 201-202°C.

Elemental Analysis: Calc'd for $C_{13}H_{10}FN_5O$ (MW 271.26): C, 57.56; H, 3.72; N, 25.82. Found:C, 57.55; H, 3.74; N, 25.82.

TLC: Ethyl acetate-hexanes (1:2)

(B) 9-(2-Fluorobenzyl)-6-methylamino-9H-purine

To a stirred slurry of lithium aluminum hydride (0.040g, 1.05 mmol) in tetrahydrofuran (20 ml) under nitrogen was added a slurry of 9-(2-fluorobenzyl)-6-formamido-9H-purine (0.203g, 0.75 mmol) in tetrahydrofuran (20 ml). The reaction was stirred at ambient temperature for 20 min and then refluxed for 2 hr. The reaction was cooled, quenched with water (3 ml) and 1N sodium hydroxide (5 ml) and then stirred for 30 min. The solids were removed by filtration and washed with water (20 ml) and ethanol (20 ml). The filtrates and washes were combined and spin evaporated in vacuo. The residue was dissolved in ethyl acetate (150 ml), washed with water (3 x 20 ml), dried ($MgSO_4$) and spin evaporated in vacuo. The white residue was recrystallized from ethyl acetate-hexanes to give 0.039g (20% of theory) of 9-(2-fluorobenzyl)-6-methylamino-9H-purine, mp 151-153°C which was identical to that prepared in Example 1.

EXAMPLE 23: 9-(2-Fluorobenzyl)-6-methylamino-9H-purine

(A) 6-Phenylthiomethylamino-9-(2-fluorobenzyl)-9H-purine

A mixture of 6-amino-9-(2-fluorobenzyl)-9H-purine (0.510g, 2.10 mmol), ethanol (15 ml), benzenethiol (0.93g, 8.48 mmol) and aqueous formaldehyde (36-38% in water) (1 ml) was refluxed with stirring for 23 hr. The reaction was spin evaporated in vacuo and dissolved in 50 ml of dichloromethane. This solution was added to silica gel 60 (15g) (40-63 µm, E. Merck No. 9385) and evaporated to dryness. The solids were introducted onto a column (4 cm x 18 cm) of silica gel 60 wetted with ethyl acetate:cyclohexane/1:1. The column was eluted with ethyl acetate:cyclohexane/2:1 using the "flash chromatography technique"[2]. The appropriate fractions were combined and evaporated to give an oil which crystallized under cyclohexane. Recrystallization from cyclohexane-ethyl acetate gave 0.307g (40% of theory) of 6-phenylthiomethylamino-9-(2-fluorobenzyl)-9H-purine, mp 135-135.5°.

Elemental Analysis: Calc'd for $C_{19}H_{16}FN_5S$ (MW 365.42): C, 62.44; H, 4.41; N, 19.17. Found:C, 62.33; H, 4.46; N, 19.16.

TLC: ethyl acetate-cyclohexane (1:1)

(B) 9-(2-Fluorobenzyl)-6-methylamino-9H-purine

A solution of 6-phenylthiomethylamino-9-(2-fluorobenzyl)-9H-purine (0.275g, 0.75 mmol), sodium borohydride (0.072g, 2.0 mmol), and 1,2-dimethoxyethane (25 ml) was refluxed for 2 hr. The cooled reaction was diluted with methanol (10 ml), acetone (3 ml), and acetic acid (0.5 ml). The volatiles were removed by spin evaporation in vacuo. The residue was dissolved in ethyl acetate (150 ml) and washed with water (30 ml), 5% sodium hydrogen carbonate (2 x 20 ml), water (20 ml), dried ($MgSO_4$) and spin evaporated in vacuo. The residue was recrystallized from ethyl acetate-cyclohexane to give 0.107g (55% of theory) of 9-(2-fluorobenzyl)-6-methylamino-9H-purine, mp 154-155°C which was identical to that prepared in Example 1.

EXAMPLE 24: 9-(2-Fluorobenzyl)-6-methylamino-9H-purine

(A) 9-(2-Fluorobenzyl)-1-methyladenium iodide

A mixture of 6-amino-9-(2-fluorobenzyl)-9H-purine (1.00g, 4.12 mmol), methyl iodide (1 ml) and dimethylformamide (10 ml) was stirred in the dark for 24 hr at ambient temperature. The reaction mixture was diluted with ethanol (40 ml) and stirred for 3 hr. The white solids were collected on a Buchner funnel and washed with ethanol. Recrystallization from water gave 0.98g (62% of theory) of 9-(2-fluorobenzyl)-1-methyladenium iodide, mp 290°C (dec).

Elemental Analysis: Calc'd for $C_{13}H_{13}FN_5I$ (MW 385.18): C, 40.53; H, 3.40; N, 18.18.
Found: C, 40.49; H, 3.41; N, 18.10.

TLC: ethyl acetate

(B) 9-(2-Fluorobenzyl)-6-methylamino-9H-purine

A mixture of 9-(2-fluorobenzyl)-1-methyladenium iodide (0.20g, 0.52 mmol) and ten percent sodium hydroxide in methanol (60 ml) was stirred at ambient temperature for 2.5 days. The reaction was spin evaporated to remove the volatiles. The residue was dispersed in ice water (100 ml) and stirred for 2 hr. The white solids were collected, washed with water and dried in vacuo to give 0.11g (83% of theory) of 9-(2-fluorobenzyl)-6-methylamino-9H-purine, mp 151-153°C which was identical to that prepared in example 1.

EXAMPLE 25: 9-(2-Fluorobenzyl)-6-methylamino-9H-purine

(A) 9-(2-Fluorobenzyl)-6-(N-methylformamido)-9H-purine

A solution of 6-dimethylamino-9-(2-fluorobenzyl)-9H-purine (1.0g, 3.68 mmol) in 50% aqueous acetic acid (20 ml) was stirred at ambient temperature while three portions of potassium permanganate (0.582g, 3.68 mmol; 0.290g, 1.84 mmol; and 0.290g, 1.84 mmol) were added at 20 min intervals. After an additional 30 minutes the dark slurry was diluted with methanol (60 ml), filtered through a Celite 545 pad and spin evaporated in vacuo. The dark residue was leached with ethyl acetate (150 ml) and filtered to give a light yellow solution

which was added to Silica Gel 60 (40-63 μm, E. Merck No. 9385). This mixture was spin evaporated in vacuo and the residual solids were added to a column (2.5cm x 20cm) of Silica Gel 60 (40-63 μm. E. Merck No. 9385) wetted with ethyl acetate. The column was eluted with ethyl acetate using the "flash chromatography technique"[2]. The appropriate fractions were combined and spin evaporated in vacuo. The white, solid residue was recrystallized from ethyl acetate-hexanes to give 0.282g (27% of theory) of 9-(2-fluorobenzyl)-6-(N-methylformamido)-9H-purine, mp 142.5 -143.5°C.

Elemental Analysis: Calc'd for $C_{14}H_{12}FN_5O$ (MW 285.28): C, 58.94; H, 4.24; N, 24.55.

Found: C, 58.84; H, 4.26; N, 24.50

(B) 9-(2-Fluorobenzyl)-6-methylamino-9H-purine

A solution of 9-(2-fluorobenzyl)-6-(N-methylformamido)-9H-purine (0.20g, 0.70 mmol), methanol (10 ml) and 1N aqueous sodium hydroxide (5 ml) was stirred at ambient temperature for 2.5 hr. The reaction mixture was spin evaporated to remove the volatiles, and the residue was dispersed in water (10 ml) and stirred for 15 hr. The white solids were collected, washed with water and recrystallized from ethyl acetate-cyclohexane to give 0.156g (86% of theory) of 9-(2-fluorobenzyl)-6-methylamine-9H-purine, mp 153-154° which was identical to that prepared in example 1.

EXAMPLE 26: 9-(2-Fluorobenzyl)-6-methylamino-9H-purine

(A) 6-(Dimethylaminomethyleneamino)-9-(2-fluorobenzyl)-9H-purine

A mixture of 6-amino-9-(2-fluorobenzyl)-9H-purine (2.00g, 8.22 mmol) and dimethylformamide dimethylacetal (30 ml) was refluxed with stirring for 1 hr. The reaction mixture was cooled, and the white solids were collected and washed with cyclohexane to give 1.80g (73% of theory) of 6-(dimethylaminomethyleneamino)-9-(2-fluorobenzyl)-9H-purine, mp 210-212°C.

Elemental Analysis: Calc'd for $C_{15}H_{15}FN_6$ (MW 298.32): C, 60.39; H, 5.07; N 28.17.

Found: C, 60.29; H, 5.11; N,28.09.

TLC: ethanol-ethyl acetate (1:3)

MRH/MHD/19th February 1985

(B) 9-(2-Fluorobenzyl)-6-methylamino-9H-purine

A solution of 6-(dimethylaminomethyleneamino)-9-(2-fluorobenzyl)-9H-purine (0.150g, 0.50 mmol), dimethylsulfoxide (6 ml), methanol (six drops) and sodium borohydride (0.045g, 1.19 mmol) was heated with stirring at 100°C for 2 hr. The reaction solution was cooled and diluted with ice water (25 ml) and stirred on an ice bath for 1 hr. The white solids were collected and washed with water. Recrystallization from ethyl acetate-cyclohexane gave 0.084g (65% of theory) of 9-(2-fluorobenzyl)-6-methylamino-9H-purine, mp 151-153°C which was identical to that prepared in Example 1.

EXAMPLES 27 and 28

The compounds of examples 27 and 28 were prepared according to a method directly analogous to that described in Example 18.

| Example | Compound | m.p. (°C) |
|---|---|---|
| 27 | 6-Amino-9-(2,6-difluorobenzyl)-9H-purine hydrochloride | 276-280 (eff.) |
| 28 | 6-Amino-9-(2,5-difluorobenzyl)-9H-purine hydrochloride | 298-301 (eff.) |

EXAMPLE 29: 9-(2-Fluorobenzyl)-6-hydroxymethylamino-9H-purine

A mixture of 6-amino-9-(3-fluorobenzyl)-9H-purine (0.847 g, 3.48 mmol), aqueous formaldehyde (36-38%) (0.282 g, 3.48 mmol), 1 N aqueous sodium hydroxide (5 drops), and tetrahydrofuran (3.0 ml) was stirred at ambient temperature for 21 hours. The reaction was spin evaporated in vacuo, and the residue was slurried in water (5ml) to give the product, 0.802 g (84% of theory), mp 120° (resolidified and remelted at 222°C). Recrystallisation from ethyl acetate/hexanes gave the analytical sample of 9-(2-fluorobenzyl)-6-hydroxymethylamino-9H-purine which was contaminated with 6-amino-9-(2-fluorobenzyl)-9H-purine (approximately 0.030 eq) and bis(9-(2-fluorobenzyl)-9H-purinylamino)-methane (approximately 0.119 eq).

Elemental Analysis: Calcd for $C_{13}H_{12}FN_5O$, 0.030 $(C_{12}H_{10}FN_5)$·0.119 $(C_{25}H_{20}F_2N_{10})$·0.2 $H_2O$ (MW 299.797): C, 57.03; H, 4.43; N, 25.81. Found: C, 57.06; H, 4.45; N, 25.61.

HPLC: Supelco $C_1$ reverse phase column, 0.01 M $NH_4H_2PO_4$ in $H_2O$/Methanol (65:35), UV detection at 256 nm.

MRH/MHD/19th February 1985

EXAMPLE 30: 9-(2-Fluorobenzyl)-6-(N-methoxymethyl-N-methylamino)-9H-purine

A mixture of 9-(2-fluorobenzyl)-6-methylamino-9H-purine (5.0 g, 19.4 mmol), sodium hydroxide (0.80 g, 20 mmol), water (1ml), and tetrahydrofuran (100ml) was heated to dissolve the solids. The stirred reaction was cooled to 25°C and aqueous formaldehyde (36-38%) (10ml) was added. The reaction was stirred for 1 hour and additional aqueous formaldehyde (10ml) was added. The reaction was heated at 50°C for 10 minutes and then cooled to 25° over a 1.5 hour period. Additional sodium hydroxide (0.50 g, 12.5 mmol) was added and stirring was continued for 5 hours. The pH of the reaction was adjusted to 7 with 12 N hydrochloric acid and then the volatiles were spin evaporated in vacuo to give 6.2 g of an oily mixture of 9-(2-fluorobenzyl-6-methylamino-9H-purine and 9-(2-fluorobenzyl)-6-(N-methoxymethyl)-N-methylamino)-9H-purine. The oil was combined with the product from a similar reaction (7.9 g total) and dissolved in a solution of hydrochloric acid (12 N) (20 ml) in methanol (100ml). After 1 hour the reaction was filtered and neutralised with a solution of potassium hydroxide (87%) (2 g, 30.3 mmol) in methanol (10ml). This solution was spin evaporated in vacuo, the residual oil was dissolved in ethyl acetate and added to Silica Gel 60 (E. Merck, No. 9385, 40-63 µm). The mixture was spin evaporated in vacuo, and the residual solids were added to a column (5 cm x 20 cm) of Silica Gel 60 (E. Merck No. 9385, 40-63 µm) wetted with ethyl acetate:hexane/1:1. The column was eluted with ethyl acetate:hexane/1:1 using the "flash chromatography technique" (W.C. Still, M. Kahn, and A. Mitra, J. Org. Chem., 43, 2923 (1978)). The appropriate fractions were combined, spin evaporated in vacuo, and the residual oil was crystallised from pentane to give 2.4 g (23% of theory) of 9-(2-fluorobenzyl)-6-(N-methoxymethyl-N-methylamino)-9H-purine, mp 64.5-65.0°.

Elemental analysis: Calcd for $C_{15}H_{16}FN_5O$ (MW 301.328): C, 59.79%: H, 5.35%; N, 23.24%. Found: C, 53.84%; H, 5.36%; N, 23.20%.
TLC: Ethyl acetate.

EXAMPLE 31: 9-(2-Fluorobenzyl)-6-methoxymethylamino-9H-purine

To a stirred mixture of 9-(2-fluorobenzyl)-6-hydroxymethylamino-9H-purine (3.0g, 11.0 mmol) and methanol (60ml) was added 12 N hydrochloric acid (2.0 ml). After 1 hour the solution was neutralised by the dropwise addition of a solution of potassium hydroxide (87%) (2.0g, 30.3 mmol) in methanol (10ml). The reaction was cooled and the resultant solids were collected by filtration. The filtrates were spin evaporated *in vacuo* to give a solid. The combined solids were

dissolved in ethyl acetate, added to Silica Gel 60 (E. Merck No. 9385, 40-63 µm) and spin evaporated in vacuo. The solids were added to a column (5cm x 20cm) of Silica Gel 60 (E. Merck No. 9385, 40-63 µm) wetted with a 1:1 mixture of ethyl acetate/hexanes and eluted using the "flash chromatography technique" (W.C. Still, M. Kahn, and A. Mitra, J. Org. Chem., 43, 2923 (1978)). The appropriate fractions were combined, spin evaporated in vacuo and recrystallised from ethyl acetate/hexanes to yield 2.1 g (66.4% of theory) of 9-(2-fluorobenzyl)-6-methoxy-methylamino-9H-purine, mp 151.0-151.5°C. Recrystallisation from methanol gave 1.4g (44.4% of theory) of the analytical sample, mp, 151.0-151.5°C.

Elemental Analysis: Calcd for $C_{14}H_{14}FN_5O$ (MW 287.301): C, 58.53; H, 4.91; N, 24.38. Found: C, 58.44; H, 4.96; N, 24.36.
TLC: Ethyl Acetate.

EXAMPLE 32: TABLET

| | |
|---|---|
| 9-(2-Fluorobenzyl)-6-methylamino-9H-purine hydrochloride | 25 mg |
| Corn Starch | 45 mg |
| Polyvinylpyrrolidone | 6 mg |
| Stearic Acid | 12 mg |
| Magnesium Stearate | 2 mg |
| Lactose | q.s. to 300 mg |

The 9-(2-fluorobenzyl)-6-methylamino-9H-purine hydrochloride was finely ground and intimately mixed with the powdered excipients lactose and corn starch. The powders were wetted with a solution of polyvinylpyrrolidone dissolved in purified water and denatured alcohol to form granules. The granules were dried and mixed with the powdered stearic acid and magnesium stearate. The formulation was then compressed into tablets weighing approximately 300 mg each.

In Examples 33 through 36 the active ingredient is 9-(2-fluorobenzyl)-6-methylamino-9H-purine hydrochloride.

EXAMPLE 33

CAPSULE

| | |
|---|---|
| Active Ingredient | 25 mg |
| Corn Starch | 45 mg |
| Stearic Acid | 12 mg |
| Lactose | q.s. to |

The finely ground active ingredient was mixed with the powdered excipients lactose and corn starch, and stearic acid and filled into hard-shell gelatin capsules.

EXAMPLE 34

SUPPOSITORY

| | |
|---|---|
| Active Ingredient | 25 mg |
| Cocoa Butter | 1,975 mg |

The cocoa butter was heated to melting and the active ingredient was dispersed by thorough mixing. The mass was then formed into suppositories weighing approximately 2,000 mg each.

## EXAMPLE 35
## INJECTION

| | |
|---|---|
| Active Ingredient | 25 mg |
| Sodium Chloride | 0.9% |
| Preservative | 0.1% |
| Hydrochloric Acid or Sodium Hydroxide | as needed for pH adjustment |
| Water for Injection | q.s. to 2-3 ml |

The active ingredient, sodium chloride, and preservative were idssolved in a portion of the water for injection. The pH of the solution was adjusted with hydrochloric acid or sodium hydroxide. Water for injection was added to final volume and the solution was thoroughly mixed. The solution was sterilized by filtration through a 0.22 micrometer membrane filter and aseptically filled into sterile containers.

## EXAMPLE 36
## SYRUP

| | |
|---|---|
| Active Ingredient | 15 mg |
| Glycerin | 500 mg |
| Sucrose | 3,500 mg |
| Flavouring Agent | q.s. |
| Colouring Agent | q.s. |
| Preserving Agent | 0.1% |
| Purified Water | q.s. to 5 ml |

The active ingredient and sucrose were dissolved in the glycerin and a portion of the purified water. The preserving agent was dissolved in another portion of hot purified water, and then the colouring agent was added and dissolved. The two solutions were mixed and cooled before the flavouring agent was added. Purified water was added to final volume. The resulting syrup was thoroughly mixed.

Claims

1. A compound of the formula (III):

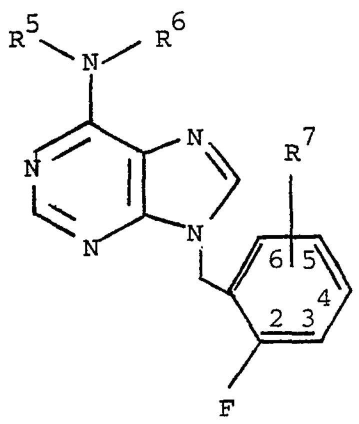

(III)

or an acid addition salt thereof, wherein $R^5$ is hydrogen, methyl or ethyl; $R^6$ is hydrogen, methyl, hydroxymethyl, methoxymethyl, ethyl, n-propyl, isopropyl, cyclopropyl, cyclopentyl, or cyclopropylmethyl and $R^7$ is hydrogen or fluorine attached to the 6- or 5- position of the phenyl ring.

2. A compound of the formula (IIIA):

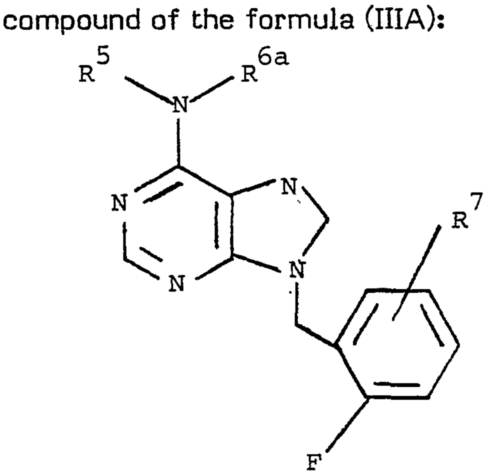

(IIIA)

or an acid addition salt thereof, wherein $R^{6a}$ is hydrogen, methyl, ethyl, n-propyl, isopropyl, cyclopropyl, cyclopentyl or cyclopropylmethyl and $R^5$ and $R^7$ are as defined in claim 1.

3. A compound of the formula (IV):

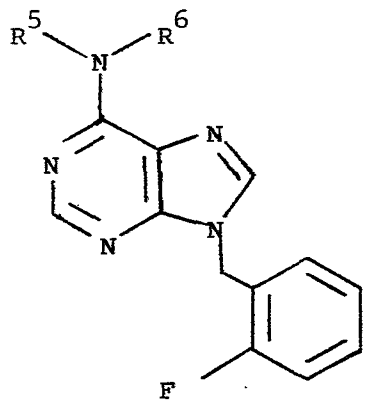

(IV)

or an acid addition salt thereof, wherein $R^5$ and $R^6$ are as defined in claim 1.

4. A compound according to claim 3 wherein $R^5$ is hydrogen and $R^6$ is hydrogen or methyl.

5. A compound selected from the group comprising:

9-(2-Fluorobenzyl)-6-methylamino-9H-purine,
6-dimethylamino-9-(2-fluorobenzyl)-9H-purine,
6-cyclopropylamino-9-(2-fluorobenzyl)-9H-purine,
6-ethylamino-9-(2-fluorobenzyl)-9H-purine,
9-(2-fluorobenzyl)-6-isopropylamino-9H-purine,
6-cyclopropylmethylamino-9-(2-fluorobenzyl)-9H-purine,
9-(2-fluorobenzyl)-6-(N-methylcyclopropylamino)-9H-purine,
9-(2-fluorobenzyl-6-(N-methylethylamino)-9H-purine,
9-(2-fluorobenzyl)-6-propylamino-9H-purine,
6-diethylamino-9-(2-fluorobenzyl)-9H-purine,
6-cyclopentylamino-9-(2-fluorobenzyl)-9H-purine,
6-cyclopropylamino-9-(2,6-difluorobenzyl)-9H-purine,
9-(2,6-difluorobenzyl)-6-dimethylamino-9H-purine,
9-(2,5-difluorobenzyl)-6-methylamino-9H-purine,
6-amino-9-(2-fluorobenzyl)-9H-purine,
6-amino-9-(2,6-difluorobenzyl)-9H-purine,
6-amino-9-(2,5-difluorobenzyl)-9H-purine,
9-(2-fluorobenzyl)-6-hydroxymethylamino-9H-purine,
9-(2-fluorobenzyl)-6-(N-methoxymethyl-N-methylamino)-9H-purine,
9-(2-fluorobenzyl)-6-methoxymethylamino-9H-purine,
and acid addition salts thereof.

6. 9-(2-fluorobenzyl)-6-methylamino-9H-purine or an acid addition salt thereof.

7. 6-amino-9-(2-fluorobenzyl)-9H-purine or an acid addition salt thereof.

8. A compound of the formula (III), for use in the treatment of CNS disorders.

9. A pharmaceutical formulation comprising a compound of the formula (III) in admixture with a pharmaceutically acceptable carrier.

10. A process for the preparation of a compound of the formula (III) which process comprises:

a) the reaction of an amine $HNR^5R^6$ with a compound of the formula ( V):

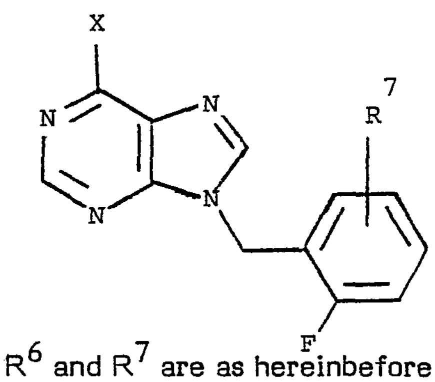

(V)

wherein $R^5$, $R^6$ and $R^7$ are as hereinbefore defined and X is a leaving group;

b) the reaction of a compound of the formula (VI) with a compound of the formula (VII):

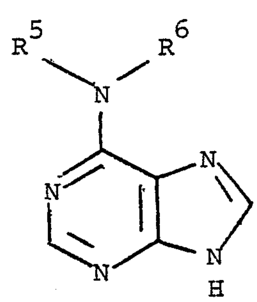

(VI)

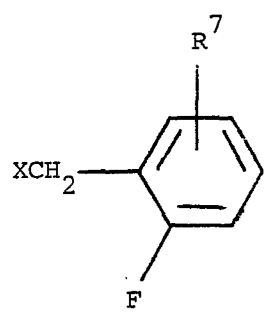

(VII)

wherein $R^5$, $R^6$, $R^7$ and X are as hereinbefore defined;

c) the reduction of a compound of the formula (VIII):

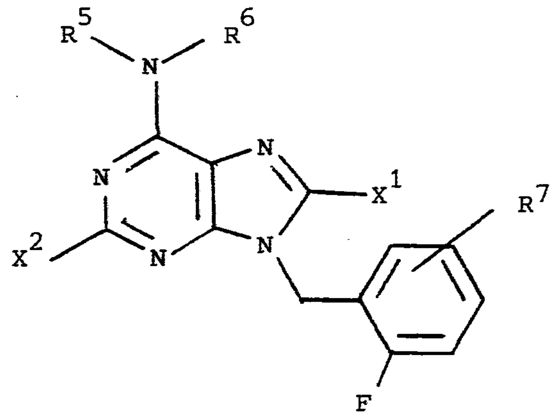

(VIII)

wherein $R^5$, $R^6$ and $R^7$ are as hereinbefore defined and $X^1$ and $X^2$ are the same or different and each is hydrogen, halogen, $C_{1-4}$ alkylthio or an alkylthio group provided that at least one of $X^1$ and $X^2$ is other than hydrogen;

d) when it is required to form a compound of the formula (III) wherein $R^5$ is hydrogen, methyl or ethyl and $R^6$ is hydrogen, methyl, ethyl, propyl or cyclopropylmethyl, such that at least one of $R^5$ and $R^6$ is other than hydrogen, the reduction of a compound of the formula (IX):

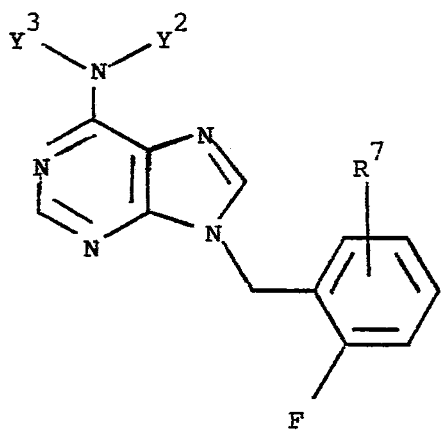

(IX)

wherein $R^7$ is as hereinbefore defined; $Y^2$ is a group $R^6$, $-(CH_2)n\ SR^8$ or $COR^9$ wherein $R^8$ is $C_{6-10}$ aryl, $C_{1-4}$ alkyl or $C_{1-10}$ arylalkyl, n = 1-3 and $R^9$ is hydrogen, cyclopropyl, methyl or ethyl and $Y^3$ is a group $R^5$, $-(CH_2)m\ SR^{8a}$ or $-COR^{10}$ wherein m is 1 or 2, $R^{8a}$ is $C_{1-4}$ alkyl and $R^{10}$ is hydrogen or methyl provided that $Y^2$ is not $R^6$ when $Y^3$ is a group $R^5$;

e) when it is required to form a compound of the formula (III) wherein $R^5$ is hydrogen and $R^6$ is methyl, ethyl or propyl, the base catalysed

rearrangement of a compound of the formula (X) or its tautomeric salt (XI):

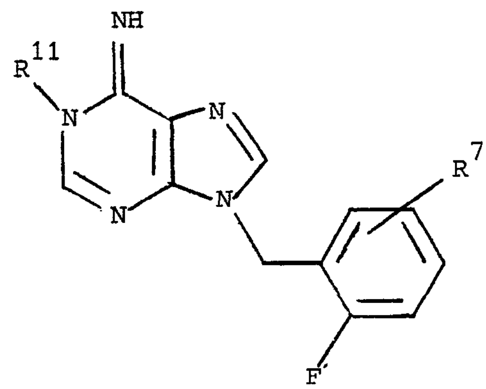

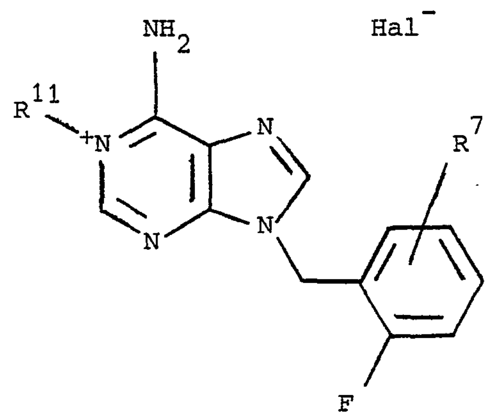

(X) (XI)

wherein $R^{11}$ may be methyl, ethyl or propyl and $R^7$ is as hereinbefore defined;

f) when it is required to form a compound of the formula (III) wherein $R^5$ is hydrogen, (i) the base catalysed hydrolysis of a compound of the formula (XII);

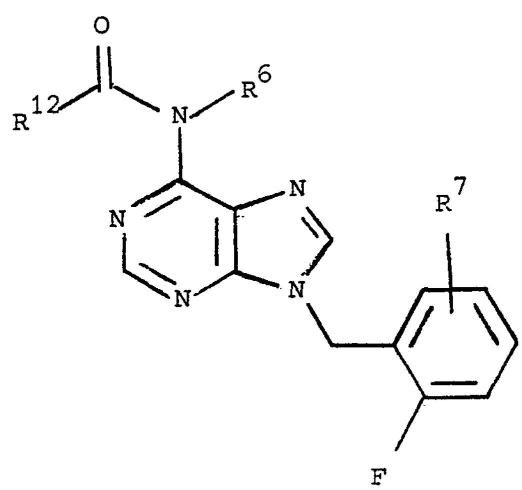

(XII)

wherein $R^6$ and $R^7$ are as hereinbefore defined and $R^{12}$ is hydrogen, $C_{1-10}$ alkyl, $C_{6-10}$ aryl or $C_{1-14}$ arylalkyl;

(ii) the reduction of a compound of the formula (XIII):

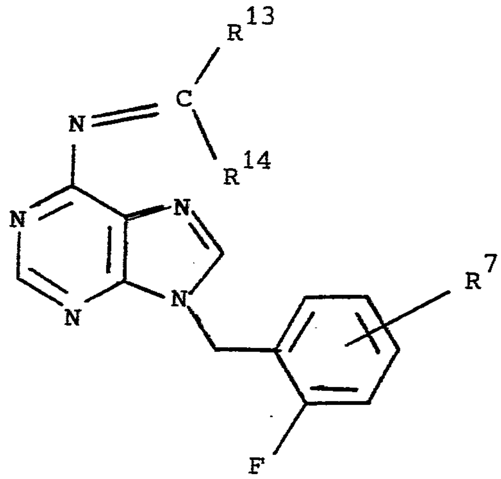

(XIII)

wherein $R^7$ is as hereinbefore defined and $R^{13}$ is a $C_{1-4}$ dialkylamino group and $R^{14}$ is hydrogen or a methyl, ethyl or cyclopropyl group;

and thereafter optionally converting one compound of the formula (III) into another compound of the formula (III).

11. Novel Intermediates of the formulae (V) and (VIII) - (XIII).

12. The use of a compound of the formula (III) for manufacture of a medicament for the treatment of CNS disorders.

MH/MHD/7th March 1985

Claims

1. A method for the preparation of a compound of the formula (III):

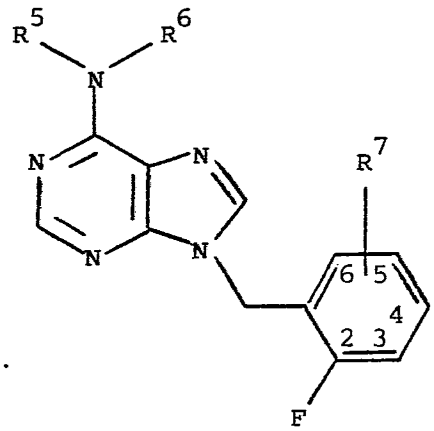

(III)

or an acid addition salt thereof, wherein $R^5$ is hydrogen, methyl or ethyl; $R^6$ is hydrogen, methyl, hydroxymethyl, methoxymethyl, ethyl, n-propyl, isopropyl, cyclopropyl, cyclopentyl, or cyclopropylmethyl and $R^7$ is hydrogen or fluorine attached to the 6- or 5- position of the phenyl ring, which method comprises:

a) the reaction of an amine $HNR^5R^6$ with a compound of the formula (V):

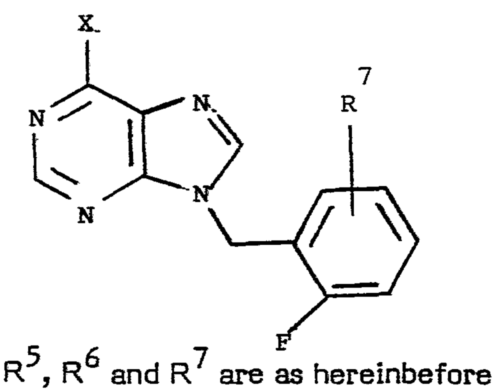

(V)

wherein $R^5$, $R^6$ and $R^7$ are as hereinbefore defined and X is a leaving group;

b) the reaction of a compound of the formula (VI) with a compound of the formula (VII):

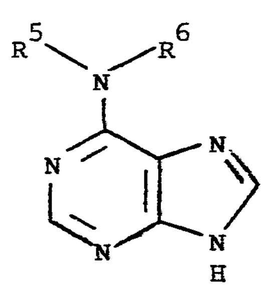

(VI)

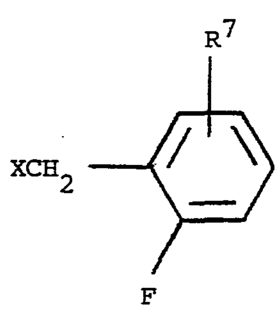

(VII)

wherein $R^5$, $R^6$, $R^7$ and X are as hereinbefore defined;

c) the reduction of a compound of the formula (VIII):

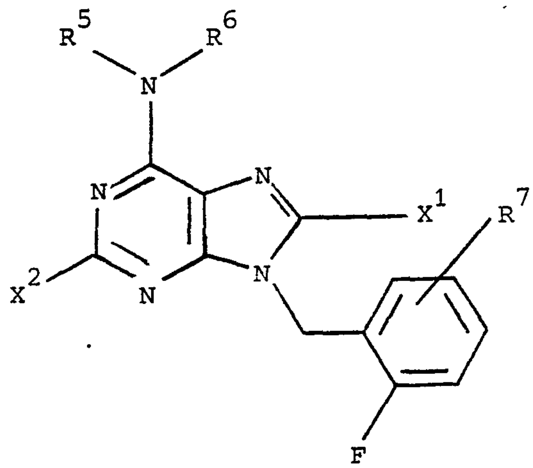

(VIII)

wherein $R^5$, $R^6$ and $R^7$ are as hereinbefore defined and $X^1$ and $X^2$ are the same or different and each is hydrogen, halogen, $C_{1-4}$ alkylthio or an alkylthio group provided that at least one of $X^1$ and $X^2$ is other than hydrogen;

d) when it is required to form a compound of the formula (III) wherein $R^5$ is hydrogen, methyl or ethyl and $R^6$ is hydrogen, methyl, ethyl, propyl or cyclopropylmethyl, such that at least one of $R^5$ and $R^6$ is other than hydrogen, the reduction of a compound of the formula (IX):

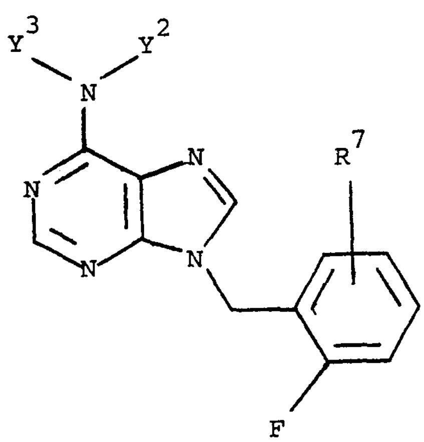

(IX)

wherein $R^7$ is as hereinbefore defined; $Y^2$ is a group $R^6$, $-(CH_2)n$ $SR^8$ or $COR^9$ wherein $R^8$ is $C_{6-10}$ aryl, $C_{1-4}$ alkyl or $C_{1-10}$ arylalkyl, n = 1-3 and $R^9$ is hydrogen, cyclopropyl, methyl or ethyl and $Y^3$ is a group $R^5$, $-(CH_2)m$ $SR^{8a}$ or -$COR^{10}$ wherein m is 1 or 2, $R^{8a}$ is $C_{1-4}$ alkyl and $R^{10}$ is hydrogen or methyl provided that $Y^2$ is not $R^6$ when $Y^3$ is a group $R^5$;

e) when it is required to form a compound of the formula (III) wherein $R^5$ is hydrogen and $R^6$ is methyl, ethyl or propyl, the base catalysed rearrangement of a compound of the formula (X) or its tautomeric salt (XI):

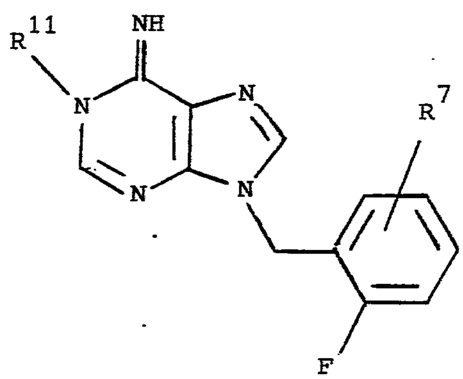

(X)

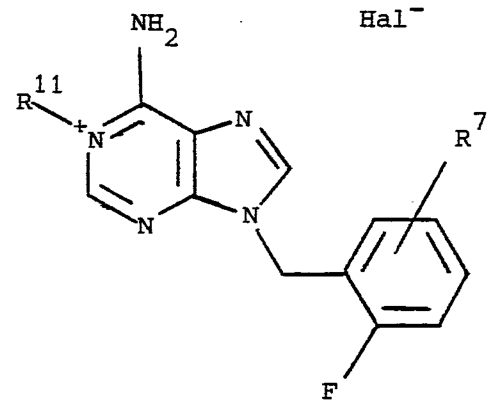

(XI)

wherein $R^{11}$ is methyl, ethyl or propyl and $R^7$ is as hereinbefore defined;

f) when it is required to form a compound of the formula (III) wherein $R^5$ is hydrogen, (i) the base catalysed hydrolysis of a compound of the formula (XII);

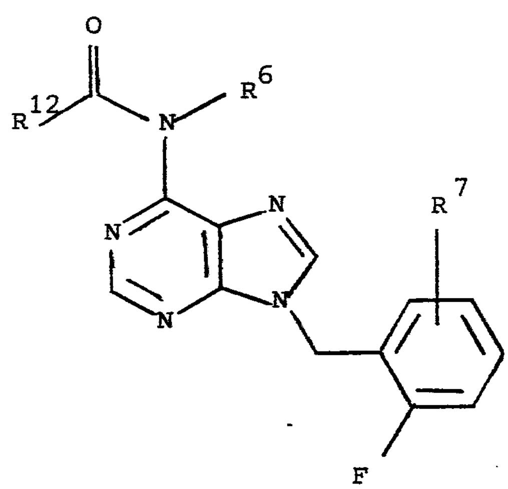

(XII)

wherein $R^6$ and $R^7$ are as hereinbefore defined and $R^{12}$ is hydrogen, $C_{1-10}$ alkyl, $C_{6-10}$ aryl or $C_{1-14}$ arylalkyl;

(ii) the reduction of a compound of the formula (XIII):

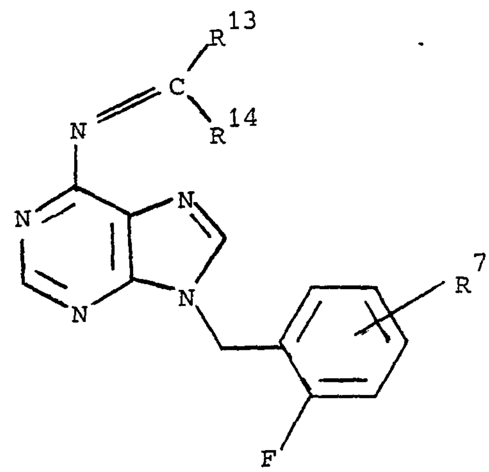

(XIII)

wherein $R^7$ is as hereinbefore defined and $R^{13}$ is a $C_{1-4}$ dialkylamino group and $R^{14}$ is hydrogen or a methyl, ethyl or cyclopropyl group;

and thereafter optionally converting one compound of the formula (III) into another compound of the formula (III).

2. A process according to claim 1 for the preparation of a compound of the formula (IIIA):

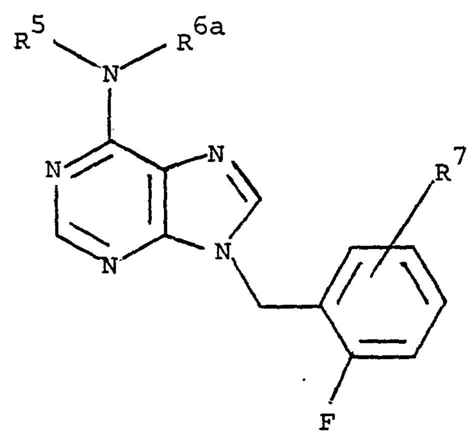

(IIIA)

or an acid addition salt thereof, wherein $R^{6a}$ is hydrogen, methyl, ethyl, n-propyl, isopropyl, cyclopropyl, cyclopentyl or cyclopropylmethyl and $R^5$ and $R^7$ are as defined in claim 1.

3. A process according to claim 1 for the preparation of a compound of the formula (IV):

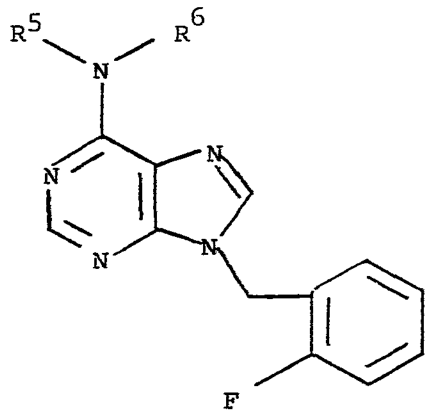

(IV)

or an acid addition salt thereof, wherein $R^5$ and $R^6$ are as defined in claim 1.

4. A process according to claim 1 for the preparation of a compound of the formula (IV), or an acid addition salt thereof wherein $R^5$ and $R^6$ are both hydrogen or wherein $R^5$ is hydrogen and $R^6$ is methyl.

5. A process according to claim 1 for the preparation of a compound chosen from the group comprising:

9-(2-Fluorobenzyl)-6-methylamino-9H-purine,
6-dimethylamino-9-(2-fluorobenzyl)-9H-purine,
6-cyclopropylamino-9-(2-fluorobenzyl)-9H-purine,
6-ethylamino-9-(2-fluorobenzyl)-9H-purine,
9-(2-fluorobenzyl)-6-isopropylamino-9H-purine,
6-cyclopropylmethylamino-9-(2-fluorobenzyl)-9H-purine,
9-(2-fluorobenzyl)-6-(N-methylcyclopropylamino)-9H-purine,
9-(2-fluorobenzyl-6-(N-methylethylamino)-9H-purine,
9-(2-fluorobenzyl)-6-propylamino-9H-purine,
6-diethylamino-9-(2-fluorobenzyl)-9H-purine,
6-cyclopentylamino-9-(2-fluorobenzyl)-9H-purine,
6-cyclopropylamino-9-(2,6-difluorobenzyl)-9H-purine,
9-(2,6-difluorobenzyl)-6-dimethylamino-9H-purine,
9-(2,5-difluorobenzyl)-6-methylamino-9H-purine,
6-amino-9-(2-fluorobenzyl)-9H-purine,
6-amino-9-(2,6-difluorobenzyl)-9H-purine,
6-amino-9-(2,5-difluorobenzyl)-9H-purine,
9-(2-fluorobenzyl)-6-hydroxymethylamino-9H-purine,
9-(2-fluorobenzyl)-6-(N-methoxymethyl-N-methylamino)-9H-purine,
9-(2-fluorobenzyl)-6-methoxymethylamino-9H-purine,
and acid addition salts thereof.

MH/MHD/19th February 1985

6. A process according to claim 1 for preparing 9-(2-fluorobenzyl)-6-methylamino-9H-purine or an acid addition salt thereof.

7. A process according to claim 1 for preparing 6-amino-9-(2-fluorobenzyl)-9H-purine or an acid addition salt thereof.

MH/MHD/19th February 1985